# EUROPÄISCHE PATENTSCHRIFT

(11) **EP 2 886 055 B1**
(45) Veröffentlichungstag und Bekanntmachung des Hinweises auf die Patenterteilung: **11.01.2017**
(21) Anmeldenummer: 14195252.3
(22) Anmeldetag: 27.11.2014
(51) Int. Cl.: A61B 5/11, A61G 7/05, F21W 131/208

(54) **Steuereinheit für ein Kranken- oder Pflegebett**
Control unit for a medical or care bed
Unité de commande pour lit de malade ou de soin

(30) Priorität: 20.12.2013 DE 202013105850 U
(43) Veröffentlichungstag der Anmeldung: 24.06.2015
(73) Patentinhaber: Wissner-Bosserhoff GmbH, 58739 Wickede (DE)
(72) Erfinder: Bernal, Carlos, 59494 Soest (DE)
(74) Vertreter: Graefe, Jörg

(56) Entgegenhaltungen:
- AU-A4- 2010 101 280
- DE-U1-212012 000 026
- US-A- 2 185 051
- US-A- 2 425 790
- US-A1- 2007 113 342
- US-A1- 2012 025 991
- DEWERT OKIN: "DUOMAT 7 Montageanleitung", , 15. Dezember 2012 (2012-12-15), XP55211239, Gefunden im Internet: URL:http://www.dewert.de/assets/products/D oppelantriebe/duomat-7/duomat_7_dewert/DUO MAT-7-D-40121.pdf [gefunden am 2015-09-04]

## Beschreibung

Die vorliegende Erfindung betrifft eine Steuereinheit für ein Kranken- oder Pflegebett gemäß dem Oberbegriff des Anspruchs 1.

Derartige Steuereinheiten sind aus dem Stand der Technik bekannt. Sie umfassen ein Detektionsmittel, das ein Detektionssignal erzeugt, wenn eine auf die Liegefläche des Betts wirkende Kraft einen Schwellwert unterschreitet. Dies hat den Zweck, dass detektiert wird, wenn eine Person das Kranken- oder Pflegebett verlässt. Es kann sich bei der Person beispielsweise um eine körperlich oder geistig geschwächte Person handeln, bei der es wünschenswert ist, dass sie sich nicht alleine außerhalb des Betts bewegt.

Das Detektionssignal kann ein für die Aufsichtsperson wahrnehmbares Signal (sichtbar und/oder hörbar) auslösen, sodass die Aufsichtsperson alarmiert wird, wenn die Person das Bett verlässt. Diese Funktion ist auch als Bett-Exit-Alarm bekannt.

Es kann jedoch einige Zeit vergehen, bis die Aufsichtsperson das Zimmer der aufgestandenen Person erreicht. Besonders in Pflegeheimen mit eine Vielzahl von Zimmern in einem Wohnbereich können je nach Auslastung der Aufsichtsperson mehrere Minuten vergehen, bis die Aufsichtsperson das relevante Zimmer erreicht. In dieser Zeit bewegt sich die aufgestandene Person alleine im Zimmer. In einem dunklen Zimmer kann die Person den Boden und eventuelle Hindernisse nicht erkennen. Dies führt zu einer erhöhten Gefährdung der Person.

Eine dem Stand der Technik entsprechende Steuereinheit für ein Kranken- oder Pflegebett mit Detektions- und Leuchtmittel ist zum Beispiel in der Gebrauchsmusterschrift DE212012000026U1 offenbart.

Es ist die Aufgabe der vorliegenden Erfindung, eine Steuereinheit zu schaffen, die die Sicherheit für eine kranke oder pflegebedürftige Person erhöht. Außerdem soll ein System mit einer solchen Steuereinheit, ein Baukastensystem mit einer solchen Steuereinheit und ein Kranken- oder Pflegebett mit einer solchen Steuereinheit geschaffen werde.

Diese Aufgabe wird durch eine Steuereinheit mit den Merkmalen des Anspruchs 1 gelöst. Ausführungsformen der Erfindung sind in den abhängigen Ansprüchen angegeben.

Es ist vorgesehen, dass die Steuereinheit ein Leuchtmittel umfasst. Das Leuchtmittel ist dazu ausgebildet, einen Bodenbereich vor dem Kranken- oder Pflegebett zu beleuchten. Dies unterscheidet das Leuchtmittel insbesondere von einem Leuchtmittel, das beispielsweise dazu ausgebildet ist, ein visuelles Signal für eine Aufsichtsperson auszugeben. Die Steuereinheit ist dazu ausgebildet, das Leuchtmittel anzuschalten, wenn das Detektionssignal erzeugt wurde.

Das Leuchtmittel beleuchtet also den Bodenbereich vor dem Kranken- oder Pflegebett, wenn die Person das Bett verlässt. Dies erhöht die Sicherheit für die Person, da zumindest der Bodenbereich vor dem Kranken- oder Pflegebett erleuchtet ist und etwaige Hindernisse für die Person sichtbar sind. Außerdem kann durch das Leuchtmittel auch der übrige Raum erhellt werden, sodass sich für die Person im gesamten Raum eine erhöhte Sicherheit ergibt.

Der Schwellwert für die Auslösung des Detektionssignals kann beispielsweise festgelegt werden, wenn die Person sich im Bett befindet. Beispielsweise kann das Detektionssignal erzeugt werden, wenn eine auf das Detektionsmittel einwirkende Kraft um einen bestimmten Wert verringert wird.

Es kann die Steuereinheit ein Gehäuse und eine elektronische Schaltung umfassen. Die elektronische Schaltung kann innerhalb des Gehäuses angeordnet sein. Das Leuchtmittel kann an einer Außenseite des Gehäuses angeordnet sein. Die elektronische Schaltung kann insbesondere komplett innerhalb des Gehäuses angeordnet sein. Die elektronische Schaltung kann insbesondere Empfangsmittel für das Detektionssignal umfassen, die elektrisch mit dem Detektionsmittel verbunden sind und dazu ausgebildet sind, das Detektionssignal zu empfangen. Außerdem kann die elektronische Schaltung Aktivierungsmittel umfassen, die dazu ausgebildet sind, das Leuchtmittel anzuschalten.

Es kann die Steuereinheit ein Befestigungselement umfassen, das an der Außenseite des Gehäuses befestigbar ist. Das Befestigungselement kann in einer ersten Ausrichtung relativ zur Steuereinheit dazu ausgebildet sein, die Steuereinheit an einem Motormodul des Kranken- oder Pflegebetts zu befestigen. Unter einem Motormodul wird dabei insbesondere ein Modul verstanden, das zumindest einen Elektromotor zur Verstellung eines Teils der Liegefläche des Kranken- oder Pflegebetts, beispielsweise des Kopfteils oder des Beinteils, umfasst. Das Motormodul kann außerdem verschiedene elektrische Anschlüsse aufweisen, an die beispielsweise ein Handbedienteil angeschlossen werden kann.

Das Befestigungselement kann insbesondere einteilig und/oder einstückig ausgebildet sein und ein Metall umfassen. Beispielsweise kann das Befestigungselement aus einem Blech gefertigt sein. Das Befestigungselement kann lösbar mit der Steuereinheit verbindbar sein. Dies kann beispielsweise über eine Schraubverbindung erreicht werden. Eine weitere Möglichkeit ist, dass das Befestigungselement an dem Gehäuse der Steuereinheit mittels einer formschlüssigen Verbindung befestigt wird.

Es kann das Befestigungselement in einer zweiten Ausrichtung relativ zur Steuereinheit dazu ausgebildet sein, die Steuereinheit an einem Energiespeichermodul des Kranken- oder Pflegebetts zu befestigten. Das Befestigungselement kann dabei in der zweiten Ausrichtung um 90° gedreht zur ersten Ausrichtung sein. Das Energiespeichermodul kann ebenfalls an dem Motormodul befestigbar sein. Somit kann die Steuereinheit entweder direkt am Motormodul oder indirekt mittels des Energiespeichermoduls am Motormodul befestigt werden. Das Energiespeichermodul kann insbesondere ein Akkumulator sein, der dazu ausgebildet ist, elektrische Energie für einen Motor des Motormoduls zu speichern.

Es kann das Befestigungselement erste Befestigungsmittel zur Befestigung an dem Energiespeichermodul und zweite Befestigungsmittel zur Befestigung am Motormodul aufweisen. Die ersten und/oder zweiten Befestigungsmittel können beispielsweise Vorsprünge sein, die dazu ausgebildet sind, in Ausnehmungen des Motormoduls und/oder des Energiespeichermoduls einzugreifen.

Es kann die Steuereinheit einen Ausgang aufweisen, über den das Detektionssignal ausgegeben werden kann. Der Ausgang kann auch Teil der elektronischen Schaltung sein. Das Detektionssignal kann somit an eine externe Vorrichtung ausgegeben werden, die beispielsweise dazu ausgebildet sein kann, ein sichtbares oder hörbares Signal für eine Aufsichtsperson auszugeben.

Es kann die Steuereinheit dazu ausgebildet sein, das Detektionssignal mit einer Zeitverzögerung auszugeben. Die Zeitverzögerung kann einstellbar sein. Die Zeitverzögerung ist vorteilhaft, wenn der Person im Bett noch zuzutrauen ist, sich für eine gewisse Zeit (beispielsweise 5, 10 oder 15 Minuten) selbstständig und alleine außerhalb des Betts zu bewegen. Hierbei ist zu beachten, dass das Detektionssignal bereits dann erzeugt wird, wenn die Person das Bett verlässt. Daher wird auch dann bereits das Leuchtmittel angeschaltet. Lediglich die Ausgabe des Detektionssignals, beispielsweise an eine externe Vorrichtung, wird verzögert.

Es kann die Steuereinheit dazu ausgebildet sein, ein Benutzersignal zu empfangen und das Leuchtmittel anzuschalten, wenn das Benutzersignal empfangen wurde. Somit lässt sich das Leuchtmittel auch unabhängig vom Detektionssignal anschalten.

Es kann die Steuereinheit einen Sender umfassen, der dazu ausgebildet ist, das Detektionssignal als drahtloses Signal auszugeben. Dies kann vorteilhaft sein, wenn das Kranken- oder Pflegebett bewegt wird, da kein Kabel eine Bewegung behindert.

Ein weiterer Aspekt betrifft ein System mit einer besagten Steuereinheit, einem Befestigungselement und einem Energiespeichermodul. Die Steuereinheit wird mittels des Befestigungselements an dem Energiespeichermodul befestigt.

Ein weiterer Aspekt betrifft ein System mit einer besagten Steuereinheit, einem Befestigungselement und einem Motormodul. Die Steuereinheit ist mittels des Befestigungselements an dem Motormodul befestigt.

Ein weiterer Aspekt betrifft ein Baukastensystem mit einer besagten Steuereinheit, einem Befestigungselement, einem Energiespeichermodul und einem Motormodul. Es ist auch möglich, dass die Steuereinheit dazu ausgebildet ist, das Detektionssignal eines Detektionsmittels zu empfangen und auszuwerten und/oder weiterzuleiten.

Unter einem Baukastensystem wird hier insbesondere verstanden, dass die Steuereinheit in einer Konfiguration direkt mit dem Motormodul verbunden werden kann. In einer anderen Konfiguration kann die Steuereinheit mit dem Energiespeichermodul verbunden werden. Das Energiespeichermodul wiederum kann mit dem Motormodul verbunden werden.

Es ist vorgesehen, dass das Befestigungselement in einer ersten Ausrichtung relativ zur Steuereinheit dazu ausgebildet ist, die Steuereinheit an dem Motormodul zu befestigen. In einer zweiten Ausrichtung relativ zur Steuereinheit ist das Befestigungselement dazu ausgebildet, die Steuereinheit an dem Energiespeichermodul zu befestigen. Das Energiespeichermodul ist an dem Motormodul befestigbar.

Auf diese Weise lassen sich einfach verschiedene Konfigurationen erzielen. Die Steuereinheit kann mit oder ohne Energiespeichermodul an dem Motormodul befestigt werden. Bei beiden Varianten wird das gleiche Befestigungselement verwendet. Dies verringert den Produktionsaufwand für verschiedene Konfigurationen.

Das Befestigungselement kann insbesondere einteilig und/oder einstückig ausgebildet sein und ein Metall umfassen. Beispielsweise kann das Befestigungselement aus einem Blech gefertigt sein. Das Befestigungselement kann lösbar mit der Steuereinheit verbindbar sein. Dies kann beispielsweise über eine Schraubverbindung erreicht werden. Eine weitere Möglichkeit ist, dass das Befestigungselement an dem Gehäuse der Steuereinheit, beispielsweise mittels einer formschlüssigen Verbindung, befestigt wird. Die Verbindung des Befestigungselements mit dem Motormodul und/oder mit dem Energiespeichermodul kann in ähnlicher Weise erfolgen.

Es kann das Befestigungselement flächenmäßig ausgebildet sein. Dies kann insbesondere bedeuten, dass eine Breite und Länge des Befestigungselements ein Vielfaches, vorzugsweise mindestens das zehnfache, der Höhe betragen.

Es kann das Befestigungselement in der ersten Ausrichtung um 90° gedreht zur zweiten Ausrichtung sein.

Außerdem kann das Befestigungselement Vorsprünge aufweisen, die dazu ausgebildet sind, in Ausnehmungen des Motormoduls einzugreifen. Das Energiespeichermodul kann ebenfalls solche Vorsprünge aufweisen.

Ein weiterer Aspeckt betrifft ein Kranken- oder Pflegebett mit einer besagten Steuereinheit

Ein weiterer Aspekt betrifft ein Kranken- oder Pflegebett mit einem besagtem System oder einem besagtem Baukastensystem.

Die Erfindung wird unter Bezugnahme auf die beiliegenden Abbildungen näher erläutert. Dabei werden für gleiche oder ähnliche Bauteile und für Bauteile mit gleichen oder ähnlichen Funktionen die gleichen Bezugszeichen verwendet. Es zeigt:
- Fig. 1: schematische Darstellungen einer Steuereinheit mit einem Befestigungselement und einem Motormodul; und
- Fig. 2: schematische Darstellungen einer Steuereinheit mit einem Befestigungselement, einem Energiespeichermodul und einem Motormodul.

Eine Steuereinheit 100 weist ein Gehäuse 101 und ein Befestigungselement 102 auf. Außerdem weist die Steuereinheit 100 ein Detektionsmittel auf, das in den Figuren nicht dargestellt ist. Das Detektionsmittel ist unterhalb einer Liegefläche eines Kranken- oder Pflegebetts angeordnet und detektiert, ob eine auf die Liegefläche wirkende Kraft einen Schwellwert unterschreitet. Das Detektionsmittel kann elektrisch mit einer elektronischen Schaltung innerhalb des Gehäuses 101 verbunden werden.

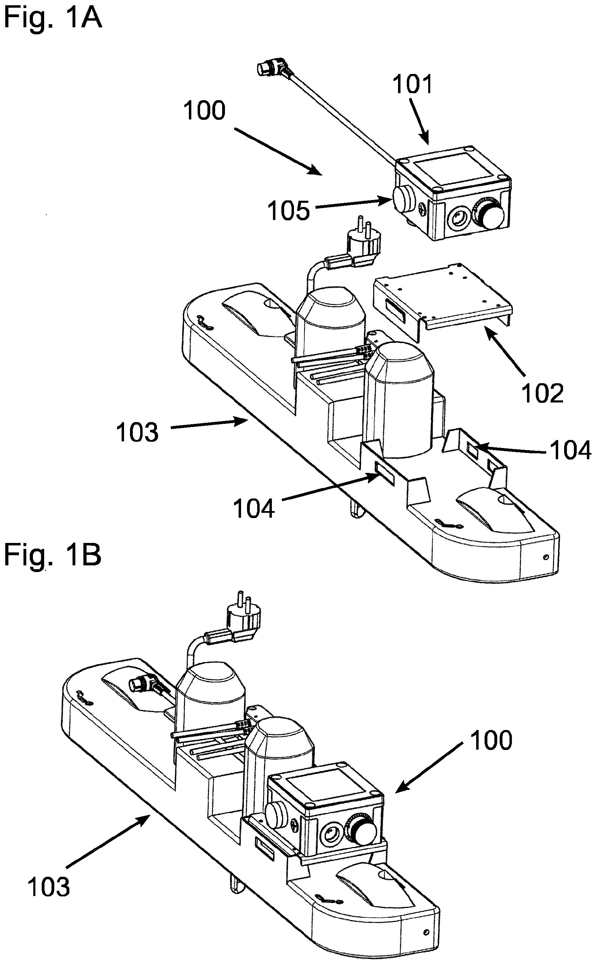

Das Befestigungselement 102 ist flächenmäßig ausgebildet und aus einem Blech gefertigt. Das Befestigungselement 102 dient in der in Figur 1 dargestellten Ausrichtung dazu, die Steuereinheit 100 an dem Motormodul 103 zu befestigen. Dafür weist das Motormodul Ausnehmungen 104 auf, in die Vorsprünge des Befestigungselements 102 eingreifen können. So entsteht eine formschlüssige Verbindung zwischen dem Befestigungselement 102 und dem Motormodul 103. Das Gehäuse 101 kann mit dem Befestigungselement 102 über Schraubverbindungen verbunden werden. Das Motormodul 103 kann an dem Kranken- oder Pflegebett angeordnet werden und umfasst zumindest einen Motor, der einen Teil der Kranken- oder Pflegebetts verstellen kann.

Außerdem ist an dem Gehäuse 101 ein Leuchtmittel 105 angeordnet. Die Steuereinheit 100 ist dazu ausgebildet, das Leuchtmittel 105 anzuschalten, wenn detektiert wurde, dass die auf die Liegefläche des Betts wirkende Kraft den Schwellwert unterschreitet.

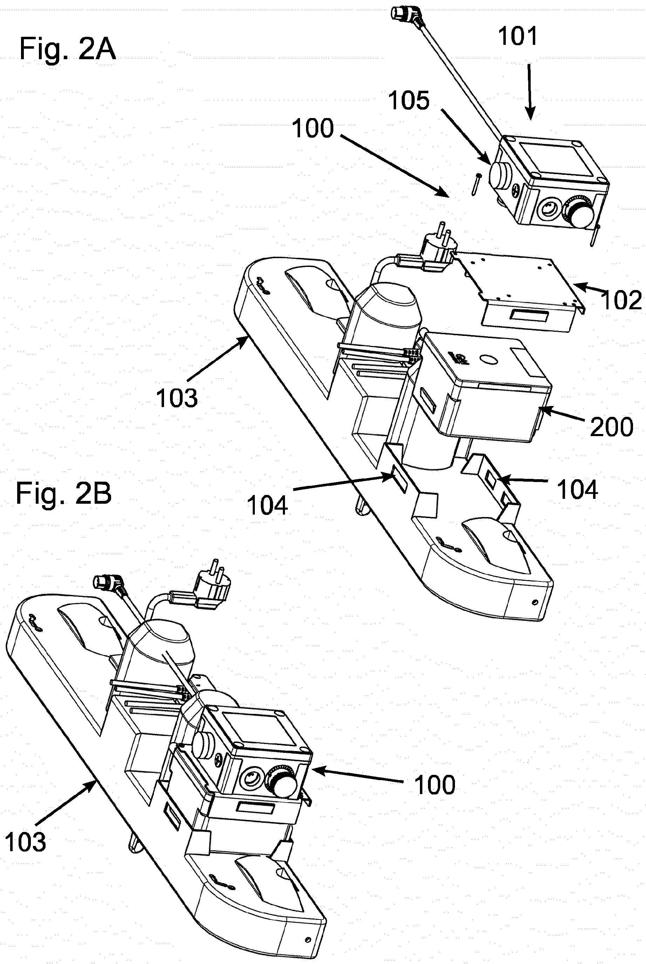

In Figur 2 ist dargestellt, wie die Steuereinheit 100 mittels des Befestigungselements 102 an einem Energiespeichermodul 200 befestigt werden kann. Dazu wird das Befestigungselement 102 relativ zur in Figur 1 dargestellten Ausrichtung um 90° gedreht. In dieser Ausrichtung kann das Gehäuse 101 mit dem Befestigungselement 102 und das Befestigungselement 102 mit dem Energiespeichermodul 200 verbunden werden. Dies kann beispielsweise über eine Schraubverbindung erfolgen.

Das Energiespeichermodul weist Vorsprünge auf, die den Vorsprüngen des Befestigungselements 102 ähneln. Die Vorsprünge greifen im montierten Zustand in die Ausnehmungen 104 des Motormoduls 103. So wird eine formschlüssige Verbindung zwischen dem Energiespeichermodul 200 und dem Motormodul 103 erreicht.

Dadurch, dass das Befestigungselement 102 in zwei Ausrichtungen verwendet werden kann, kann die Steuereinheit 100 sowohl zusammen mit dem Energiespeichermodul 200 an dem Motormodul 103 als auch alleine an dem Motormodul 103 befestigt werden. Außerdem kann sowohl das Befestigungselement 102 als auch das Energiespeichermodul 200 mit dem Motormodul 200 verbunden werden. So lässt sich mit relativ geringem Herstellungsaufwand eine hohe Flexibilität erreichen.

## Patentansprüche

1. Steuereinheit (100) für ein Kranken- oder Pflegebett mit einer Liegefläche, wobei die Steuereinheit (100) ein Detektionsmittel umfasst, das dazu ausgebildet ist, ein Detektionssignal zu erzeugen, wenn eine auf die Liegefläche wirkende Kraft einen Schwellwert unterschreitet, wobei die Steuereinheit (100) ein Leuchtmittel (105) umfasst, das dazu ausgebildet ist, einen Bodenbereich vor dem Kranken- oder Pflegebett zu beleuchten, wobei die Steuereinheit (100) dazu ausgebildet ist, das Leuchtmittel (105) anzuschalten, wenn das Detektionssignal erzeugt wurde, wobei die Steuereinheit (100) ein Gehäuse (101) und eine elektronische Schaltung umfasst, wobei die elektronische Schaltung innerhalb des Gehäuses (101) angeordnet ist und wobei das Leuchtmittel (105) an einer Außenseite des Gehäuses (101) angeordnet ist, wobei die Steuereinheit (100) ein Befestigungselement (102) umfasst, das an der Außenseite des Gehäuses (101) befestigbar ist, und dass das Befestigungselement (102) in einer ersten Ausrichtung relativ zur Steuereinheit (100) dazu ausgebildet ist, die Steuereinheit (100) an einem Motormodul (103) des Kranken- oder Pflegebetts zu befestigen,
**dadurch gekennzeichnet, dass**
das Befestigungselement (102) in einer zweiten Ausrichtung, die sich von der ersten Ausrichtung unterscheidet, relativ zur Steuereinheit (100) dazu ausgebildet ist, die Steuereinheit (100) an einem Energiespeichermodul (200) des Kranken- oder Pflegebetts zu befestigen.

2. Steuereinheit (100) nach Anspruch 1, **dadurch gekennzeichnet, dass** das Befestigungselement (102) erste Befestigungsmittel zur Befestigung an dem Energiespeichermodul (200) und zweite Befestigungsmittel zur Befestigung am Motormodul (103) aufweist.

3. System, umfassend eine Steuereinheit (100) nach einem der vorhergehenden Ansprüche, ein Befestigungselement (102) und ein Energiespeichermodul (200), wobei die Steuereinheit (100) mittels des Befestigungselements (102) an dem Energiespeichermodul (200) befestigbar ist.

4. System, umfassend eine Steuereinheit (100) nach einem der Ansprüche 1 oder 2, ein Befestigungselement und ein Motormodul (103), wobei die Steuereinheit (100) mittels des Befestigungselements (102) an dem Motormodul (103) befestigbar ist.

5. Baukastensystem, umfassend eine Steuereinheit (100) nach einem der Ansprüche 1 oder 2, ein Befestigungselement (102), ein Energiespeichermodul (200) und ein Motormodul (103), wobei das Befestigungselement (102) in einer ersten Ausrichtung relativ zur Steuereinheit (100) dazu ausgebildet ist, die Steuereinheit (100) an dem Motormodul (103) zu befestigen, wobei das Befestigungselement (102) in einer zweiten Ausrichtung relativ zur Steuereinheit (100) dazu ausgebildet ist, die Steuereinheit (100) an dem Energiespeichermodul (200) zu befestigen, und wobei das Energiespeichermodul (200) an dem Motormodul (103) befestigbar ist.

6. Baukastensystem nach Anspruch 5, **dadurch gekennzeichnet, dass** das Befestigungselement (102) flächenmäßig ausgebildet ist.

7. Baukastensystem nach einem der Ansprüche 5 oder 6, **dadurch gekennzeichnet, dass** das Befestigungselement (102) in der ersten Ausrichtung um 90° gedreht zur zweiten Ausrichtung ist.

8. Kranken- oder Pflegebett, umfassend eine Steuereinheit (100) nach einem der Ansprüche 1 oder 2.

9. Kranken- oder Pflegebett, umfassend ein System nach Anspruch 3 und/oder ein System nach Anspruch 4 oder ein Baukastensystem nach zumindest einem der Ansprüche 5 bis 7.

## Claims

1. Control unit (100) for a medical or care bed having a reclining surface, wherein the control unit (100) comprises a detection means which is designed to produce a detection signal if a force acting on the reclining surface drops below a threshold value, wherein the control unit (100) comprises an illuminating means (105) which is designed to illuminate a floor region in front of the medical or care bed, wherein the control unit (100) is designed to switch on the illuminating means (105) when the detection signal has been generated, wherein the control unit (100) comprises a housing (101) and an electronic circuit, wherein the electronic circuit is arranged within the housing (101), and wherein the illuminating means (105) is arranged on an outer side of the housing (101), wherein the control unit (100) comprises a fastening element (102) which is fastenable to the outer side of the housing (101), and in that the fastening element (102) is designed in a first orientation relative to the control unit (100) to fasten the control unit (100) to a motor module (103) of the medical or care bed, **characterized in that** the fastening element (102) is designed in a second orientation, which differs from the first orientation, relative to the control unit (100) to fasten the control unit (100) to an energy storage module (200) of the medical or care bed.

2. Control unit (100) according to Claim 1, **characterized in that** the fastening element (102) has first fastening means for fastening to the energy storage module (200) and second fastening means for fastening to the motor module (103).

3. System comprising a control unit (100) according to one of the preceding claims, a fastening element (102) and an energy storage module (200), wherein the control unit (100) is fastenable to the energy storage module (200) by means of the fastening element (102).

4. System comprising a control unit (100) according to either of Claims 1 and 2, a fastening element and a motor module (103), wherein the control unit (100) is fastenable to the motor module (103) by means of the fastening element (102).

5. Modular system comprising a control unit (100) according to either of Claims 1 and 2, a fastening element (102), an energy storage module (200) and a motor module (103), wherein the fastening element (102) is designed in a first orientation relative to the control unit (100) to fasten the control unit (100) to the motor module (103), wherein the fastening element (102) is designed in a second orientation relative to the control unit (100) to fasten the control unit (100) to the energy storage module (200), and wherein the energy storage module (200) is fastenable to the motor module (103).

6. Modular system according to Claim 5, **characterized in that** the fastening element (102) is of planar design.

7. Modular system according to either of Claims 5 and 6, **characterized in that** the fastening element (102) in the first orientation is rotated by 90° with respect to the second orientation.

8. Medical or care bed comprising a control unit (100) according to either of Claims 1 and 2.

9. Medical or care bed comprising a system according to Claim 3 and/or a system according to Claim 4 or a modular system according to at least one of Claims 5 to 7.

## Revendications

1. Unité de commande (100) pour un lit médicalisé ou lit thérapeutique avec une surface de couchage, l'unité de commande (100) comprenant un moyen de détection qui est conçu pour délivrer un signal de détection si une force exercée sur la surface de couchage n'atteint pas une valeur seuil, l'unité de commande (100) comprenant un moyen d'éclairage (105) qui est conçu pour éclairer une zone du sol à l'avant du lit médicalisé ou lit thérapeutique, l'unité de commande (100) étant conçue pour allumer le moyen d'éclairage (105) lorsque le signal de détection a été délivré, l'unité de commande (100) comprenant un boîtier (101) et un circuit électronique, le circuit électronique étant placé à l'intérieur du boîtier (101) et le moyen d'éclairage (105) étant placé sur une face extérieure du boîtier (101), l'unité de commande (100) comprenant un élément de fixation (102) susceptible d'être fixé sur la face extérieure du boîtier (101) et en ce que dans une première orientation par rapport à l'unité de commande (100), l'élément de fixation (102) étant conçu pour fixer l'unité de commande (100) sur un module moteur (103) du lit médicalisé ou lit thérapeutique,
**caractérisée en ce que**
dans une deuxième orientation par rapport à l'unité de commande (100), qui se différencie de la première orientation, l'élément de fixation (102) est conçu pour fixer l'unité de commande (100) sur un module (200) accumulateur d'énergie du lit médicalisé ou lit thérapeutique.

2. Unité de commande (100) selon la revendication 1, **caractérisée en ce que** l'élément de fixation (102) comporte des premiers moyens de fixation destinés à la fixation sur le module (200) accumulateur d'énergie et des seconds moyens de fixation destinés à la fixation sur le module moteur (103).

3. Système comprenant une unité de commande (100) selon l'une quelconque des revendications précédentes, un élément de fixation (102) et un module (200) accumulateur d'énergie, l'unité de commande (100) étant susceptible d'être fixée sur le module (200) accumulateur d'énergie à l'aide de l'élément de fixation (102).

4. Système comprenant une unité de commande (100) selon l'une quelconque des revendications 1 ou 2, un élément de fixation et un module moteur (103), l'unité de commande (100) étant susceptible d'être fixée sur le module moteur (103) à l'aide de l'élément de fixation (102).

5. Système modulaire, comprenant une unité de commande (100) selon l'une quelconque des revendications 1 ou 2, un élément de fixation (102), un module (200) accumulateur d'énergie et un module moteur (103), dans une première orientation par rapport à l'unité de commande (100), l'élément de fixation (102) étant conçu pour fixer l'unité de commande (100) sur le module moteur (103), dans une deuxième orientation par rapport à l'unité de commande (100), l'élément de fixation (102) étant conçu pour fixer l'unité de commande (100) sur le module (200) accumulateur d'énergie et le module (200) accumulateur d'énergie pouvant être fixé sur le module moteur (103).

6. Système modulaire selon la revendication 5, **caractérisé en ce que** l'élément de fixation (102) est conçu à grande surface.

7. Système modulaire selon l'une quelconque des revendications 5 ou 6, **caractérisé en ce que** dans la première orientation, l'élément de fixation (102) est tourné de 90° par rapport à la deuxième orientation.

8. Lit médicalisé ou lit thérapeutique comprenant une unité de commande (100) selon l'une quelconque des revendications 1 ou 2.

9. Lit médicalisé ou lit thérapeutique comprenant un système selon la revendication 3 et/ou un système selon la revendication 4 ou un système modulaire selon au moins l'une quelconque des revendications 5 à 7.
